# EUROPEAN PATENT SPECIFICATION

(11) **EP 3 738 017 B1**
(45) Date of publication and mention of the grant of the patent: **06.04.2022**
(21) Application number: 19705169.1
(22) Date of filing: 13.02.2019
(51) Int. Cl.: G06F 3/01, G06F 3/039, A63F 13/24, A63F 13/98

(54) **DEVICE FOR INCREASING PERFORMANCE, COMFORT AND/OR PREVENTING INJURY OF A USER OF A COMPUTER MOUSE**
VORRICHTUNG ZUR ERHÖHUNG DER LEISTUNG, DES KOMFORTS UND/ODER DER VERMEIDUNG VON VERLETZUNGEN EINES BENUTZERS EINER COMPUTERMAUS
DISPOSITIF POUR AUGMENTER LA PERFORMANCE, LE CONFORT ET/OU LA PRÉVENTION D'UNE BLESSURE D'UN UTILISATEUR D'UNE SOURIS D'ORDINATEUR

(30) Priority: 13.02.2018 SE 1850153; 15.06.2018 SE 1850735; 15.10.2018 SE 1851256
(43) Date of publication of application: 18.11.2020
(73) Proprietor: Flashe Gaming Group AB, 117 71 Stockholm (SE)
(72) Inventor: ÖDMARK, Oskar, 11771 Stockholm (SE); SJÖDIN, Johannes, 590 52 Nykil (SE)
(74) Representative: AWA Sweden AB
(86) International application number: PCT/EP2019/053492
(87) International publication number: WO 2019/158554

(56) References cited:
- DE-A1-102011 116 267
- US-A1- 2015 290 494

## Description

### Technical field

The present disclosure relates to a wearable device for increasing performance, comfort and/or preventing injury of a user of a computer mouse, or the like. The device may be used as a gaming glove, i.e. a glove that is intended for use in computer gaming, or as an ergonomic implement intended for use by any computer operator.

### Background

Many computer games are controlled at least partially by a computer mouse. Especially in so-called first-person-shooter games, the mouse may be used to control the aim of the player's weapon. Such control may require a large range of movements, from minute ones to relatively large ones. Moreover, such movements often need to be performed very quickly, leading to a very high movement speed.

It is well known, that use of computer mice may lead to injuries, such as carpal tunnel syndrome, and the like. Various devices have been produced with the aim to reduce the risks associated with the use of computer mice.

US5925007A discloses a carpal cuff, which is designed to reduce the risk of injuries on users of computer mice. Similar devices are disclosed in US6082684A, WO0236050A1 and WO09059492A1.

DE102011116267 A1 discloses a computer mouse flexibly connected to a wrist support device for supporting an ergonomic, less tiring, use of the computer mouse. US2015290494 A1 discloses a wearable device worn on the forearm of a user, whereby the device provides an ergonomically correct positioning of the forearm and hand for avoiding injuries, like repetitive stress injuries, RSI, of a user, whereby the device in addition provides computer mouse functions.

There is a need for further improvements of these devices

### Summary

An object of the present disclosure is to provide an improved device for reducing the risk of injury, as well as for improving the performance and/or comfort, of computer mouse users.

The invention is defined by the appended independent claim, with embodiments being set forth in the appended dependent claims.

### Brief description of the drawings

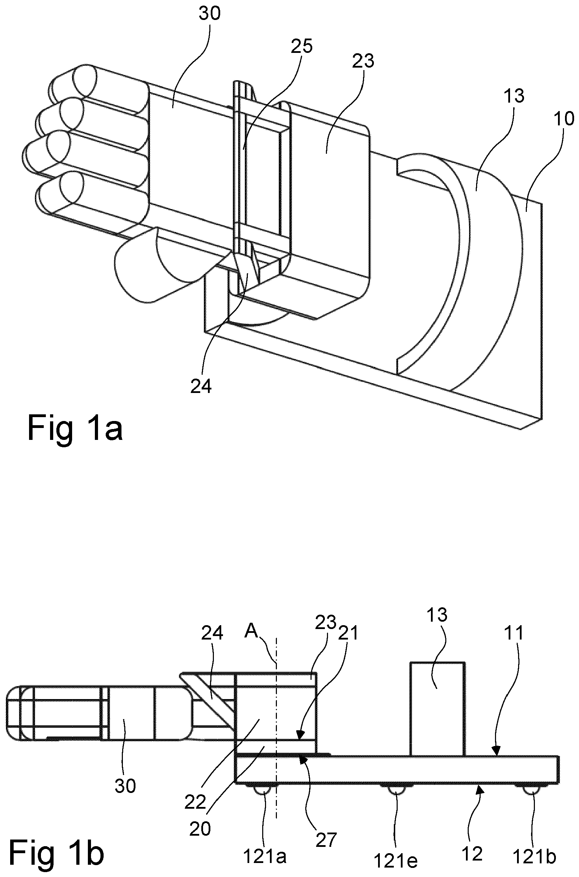

Fig 1a is a schematic perspective view of a gaming glove.
Fig 1b is a schematic side view of the gaming glove in fig. 1a.

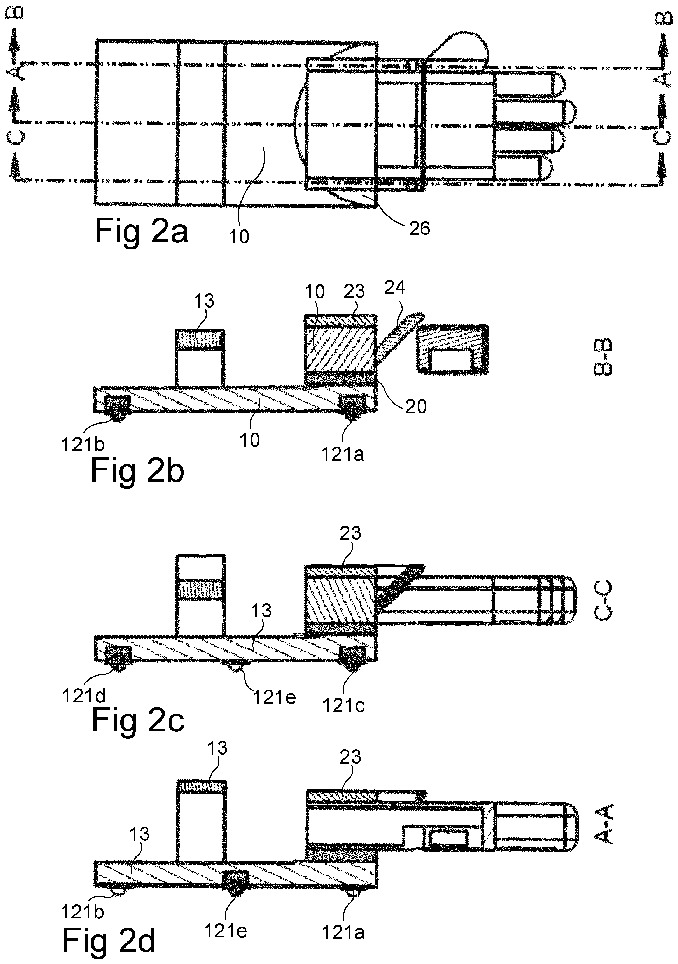

Fig 2a is a schematic top view of the gaming glove in fig. 1a.
Fig 2b-2d are schematic sectional views of the gaming glove in fig. 2a.
Fig. 3 schematically illustrates a first joint concept for providing a rotatable joint between the wrist support and the hand support.
Fig. 4 schematically illustrates a second joint concept.
Figs 5a-5b schematically illustrates a third joint concept.
Fig. 6 schematically illustrates a fourth joint concept.
Fig. 7 schematically illustrates a fifth joint concept.
Fig. 8 schematically illustrates a sixth joint concept.
Fig. 9 schematically illustrates an embodiment of a gaming glove worn by a user, as seen from below.
Fig. 10 schematically illustrates an embodiment of a gaming glove worn by a user, as seen from above.
Figs 11a-11b schematically illustrate an ergonomic support member, which may be incorporated into the gaming glove.

### Detailed description

Referring to the drawings, a gaming glove will according to the present disclosure will now be described.

The gaming glove comprises a wrist support 10, which is formed by a base member having at an underside 12 thereof, downwardly exposed bearing members 121a, 121b, 121c, 121d, 121e and an upwardly exposed face 11, which is adapted for receiving and supporting a part of a user's forearm, typically the ventral side of the user's forearm.

The wrist support 10 may be formed as a relatively flat, longitudinal member having a length of about 10-20 cm, preferably 15-20 cm, and a width of about 5-10 cm, preferably 7-10 cm.

In other embodiments, the wrist support may be designed to extend over at least 60 % of the user's radius bone, as seen along the user's arm, preferably at least 70 %, at least 80 % or at least 90 %. In some embodiments, the wrist support may extend through 100-120 % of the user's radius bone. That is, the wrist support may also provide support for the user's elbow. The gaming glove may be provided in different sizes to fit differently sized users.

The wrist support 10 may be formed of a polymeric material, such as plastic or reinforced plastic. Alternatively, a relatively light metal, such as aluminum or titanium, may be used. In any event, it may be desirable to select a material that provides minimum irritation to the user's skin. In particular embodiments, the wrist support 10, and or the hand support, may be formed of a thermoplastic polyurethane or by a thermoplastic elastomeric material.

An upper surface 11 of the wrist support may present a groove running along the longitudinal direction and adapted to receive the user' forearm.

The upper surface 11 may be provided with a surface structure and/or a material that promotes transfer of air around the user's arm. For example, the surface may have protrusions and/or depressions, ridges, grooves, etc., which allow air to flow between the user's arm and the upper surface.

As an alternative, or supplement, the upper surface 11 may be provided with a textile or fibrous material.

As yet another alternative, or supplement, the upper surface 11 may present one or more padded areas, e.g. near edges of the upper surface.

As another option, at least 50 %, preferably 60 % or 80 % of the upper surface 11 may be provided with padding to increase comfort.

Hence, the wrist support and/or the hand support may be padded, so as to provide increased comfort. Examples of material include fabrics and foamed polymer materials.

Moreover, the wrist support and/or the hand support may be formed from at least two materials, including a first material that provides rigidity and a second, less rigid material that provides comfort. For example, the first material may be a normal engineering plastic, whereas the second material may be a rubber or TPE type material. The materials may be formed separately and then bonded to each other, or they may be formed through a co-injection molding process.

A wrist fastening device 13 may be provided, configured for attaching the wrist support to the user's wrist, such that the wrist support will follow movements of the user's wrist.

The wrist fastening device 13 may comprise one or more straps, which may be resilient or non-resilient. The straps may be provided with an attachment mechanism, such as a buckle, a hook-and-loop arrangement, or the like, to allow the user to releasably and comfortably attach the wrist support to the wrist. In the case where the strap(s) is/are resilient, no attachment mechanism may be necessary.

A strap typically extends over less than 30 %, preferably less than 20 % or less than 10 %, of the length of the wrist support.

As an alternative, a resilient cuff or wristlet may be provided, which may extend over more than 30 %, preferably more than 50 % or more than 80 % of the length of the wrist support.

As yet another alternative, it is possible to use a lacing arrangement, similar to a shoe-lacing arrangement, but preferably adapted so as to enable it to be operated with one hand. An example of such an arrangement is disclosed in US2004172853A.

A more general example may be where the free ends of the lacing arrangements are twisted about a spool, followed by a locking action of the spool and the lace ends. For example, the spool may be axially movable between a first position, wherein it is rotatable for winding/unwinding the lace ends and a second position, wherein it is prevented from rotating. A bayonet type locking mechanism may be provided for locking the spool in the second position.

In embodiments using a sleeve or cuff, whether laced or otherwise possible to tension around the user's arm, it is possible to arrange such sleeve or cuff to be detachably attachable to the wrist support and optionally the hand support. This may be rendered possible by attaching the sleeve or cuff to the wrist/hand support by means of e.g. buttons, hook-and-look type fasteners or the like.

This may be particularly advantageous in embodiments where the sleeve or cuff extends over more than 30 % of a length of the user's radius bone, such as 50-120 %, preferably 50-100 % of the length.

Needless to say, the sleeve or cuff may be fixedly attached to the wrist support and optionally also to the hand support.

The underside 12 of the wrist support may present the bearing members 121a, 121b, 121c, 121d, 121e. There may be 3-10, preferably 3-7 or 4-5 bearing members 121a, 121b, 121c, 121d, 121e, arranged to define a two-dimensional support surface. For example, as illustrated, four bearings 121a, 121b, 121c, 121d may be arranged at corners of the wrist support, such that they together define a rectangle, and one or more bearings 121e may be arranged inside the rectangle, e.g. at or near its geometric center of gravity.

The bearing members 121a, 121b, 121c, 121d, 121e may be ball transfer units, or similar, each comprising a housing and a load ball, and optionally a plurality of support balls. The load ball may be made of a metallic, polymeric or ceramic material.

Alternatively, or as a supplement, the underside of the wrist support may be provided with one or more low friction surfaces, e.g. surfaces coated with a low-friction type polymer, such as PTFE. Such low friction surface may extend over all or parts of the length of the wrist support and the hand support.

At least one of the housings of the ball transfer units may be received in recesses in the underside of the wrist support.

Alternatively, at least one of the housings may be arranged on the outside of the underside of the wrist support.

As yet another option, at least one of the housings may be integrated with the wrist support. For example, a recess in the underside of the wrist support may directly receive the load ball and any support balls.

The gaming glove further comprises a hand support 20. The hand support may be made of the same material as the wrist support 10.

The hand support 10 should be configured to provide support for the carpus bones of the hand, while preferably not impeding movement of the metacarpal bones.

Hence the hand support 10 may be entirely comprised within the two-dimensional footprint provided by the wrist support 10.

Alternatively, the hand support 10 may extend slightly beyond the footprint of the wrist support. Typically, such extent will be less than 20 % of the length of the wrist support, preferably less than 10 % or less than 5 %.

An upper surface 21 of the hand support may be approximately flush with the upper surface 11 of the wrist support. To this end, the part of the wrist support 10 where the hand support 20 is connected may be recessed, so as to fit the hand support 20 and the bearing 26.

At least 50 %, preferably 60 % or 80 % of the upper surface 21 may be provided with padding to increase comfort.

The hand support 20 may be movable relative to an upwardly facing surface of the wrist support 10. In particular, the hand support may be movable relative to the wrist support along a plane that is horizontal +/- 40 degrees, preferably +/- 20 degrees or +/- 10 degrees.

Alternatively, an interface surface between the hand support and the wrist support may be part spherical.

As one example, the hand support 20 may be rotatably connected to the wrist support 10. The rotatable connection may be configured such that the hand support and the wrist support are rotatable relative to each other about an axis A that provides an angle of less than 40 degrees, preferably less than 30 degrees or less than 15 degrees, relative to a vertical direction, when the device is placed on a horizontal surface. Typically, the axis may be substantially vertical.

The axis A may be situated within the two-dimensional footprint of the wrist support, and preferably also within a two-dimensional footprint defined by the bearings 121a, 121b, 121c, 121d on which the wrist support 10 rests on the base, e.g. a table.

The rotational connection 26 may typically comprise a roller bearing, one race of which being connected to the wrist support 10 and the other race of which being connected to the hand support 20.

Alternatively, the hand support 20 may be slidable relative to the wrist support 10. The underside 27 of the hand support and the upwardly oriented bearing surface of the wrist support may be provided with a bearing arrangement, such as a glide bearing or a roller bearing.

Moreover, one of the hand support 20 and the wrist support 10 may comprise one or more guide members, which may interact with corresponding guide members on the other one of the hand support 20 and the wrist support 10. For example, a guide member may comprise a pin or a fin and a corresponding guide member may comprise a groove or a slot, which may be curved.

The guide members may control the movement direction and limit the movement, e.g. such that the hand support 20 may move and/or rotate horizontally through a limited angle relative to the wrist support 10.

Optionally, such guide members may also limit movement in the vertical direction.

Guide members as described above may be combined with the rotational connection 26 described above.

The hand support 20 may further comprise a frame 22, 23, 24, 25, which is adapted to interact laterally with the user's hand, to make sure that any sideways movement of the hand relative to the user's forearm results in a rotation about the axis A.

As illustrated, the frame may comprise one, or a pair of, vertical section(s) 22, extending upwardly from an upper surface of the hand support 20. The frame may further comprise one or two lateral sections 24, which may extend forwardly of the vertical section 22, and optionally a transversal section 25, which may extend between distal parts of the vertical sections 24 and/or between distal parts of the lateral sections 24.

The hand support 20 may further comprise a hand fastening device 30, adapted to safely attach the user's hand to the hand support 20, such that the hand support 20 will follow the user's hand movements.

The frame 22, 23, 24, 25 may be at least partially detachably connected to the hand support 20. For example, the frame may, at one lateral side thereof, be provided with a hinge and at the other lateral side thereof with a connector. Alternatively, the frame 22, 23, 24, 25 may be so flexible as to render any hinge unnecessary.

As yet another alternative, the frame 22, 23, 24, 25 may be resilient and flexible to such an extent that it returns to a closed position after having been opened, but does not need any connector.

The hand fastening device may comprise one or more of a cuff, a wristlet, a strap or a glove. In the case of a glove, possibly a fingertip-less glove.

The hand fastening device 30 may be attached to the hand support and/or to the frame 22, 23, 24, 25, if any. For example, a hand fastening device in the form of a cuff, wristlet or glove may be attached both to the hand support 20 and to the frame 22, 23, 24, 25. It may be suitable to attach such hand support to distal parts of the lateral frame members 24, to distal parts of the vertical members 22 and/or to a cross member 23, 25.

The device may be used by arranging a lower portion of a ventral side of the user's forearm to bear against the upper side 11 of the wrist support and arranging a portion of the user's hand comprising the carpus bones to bear against an upper side of the hand support.

Hence, in use, the lower part of the user's forearm rests against the wrist support and the carpus bones of the user's hand rests against the hand support.

The wrist support may be attached to the user's forearm by means of the wrist fastening device 13.

The hand support may be attached to the user's hand by means of the hand fastening device 30.

Hence, in use, the device may be securely attached to the user's arm such that the wrist support's 10 downwardly exposed bearings reduce friction against the supporting surface, which may be a table, optionally provide with a mouse mat, thus allowing the device to follow the user's arm movements.

The hand support may be securely attached to the user's hand, such that movements of the user's hand relative to the wrist are facilitated by the bearing 26 forming the rotatable connection between the hand support 20 and the wrist support 10.

The hand frame 22, 23, 24, 25 will abut, in particular, sides of the user's hand to ensure that the user's hand movements are properly transferred to the hand support.

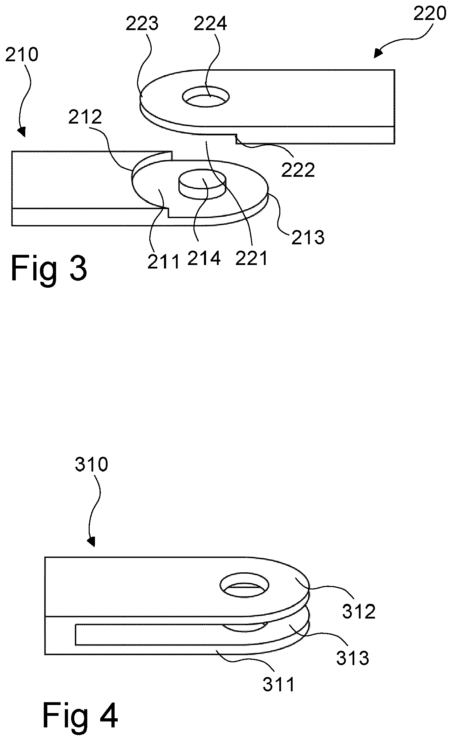

Fig. 3 schematically illustrates a first joint concept for providing a rotatable joint between the wrist support and the hand support. The joint may comprise a pair of joint members 210, 220, which may be attached to, or formed in one piece with, a respective one of the wrist support and the hand support.

The joint members may be formed so as to partially overlap each other. An area or overlap may be recessed in one or both of the joint members, such that the joint members will take up less space in the direction parallel with an axis of rotation of the joint. For example, the recesses 211, 221 may be designed so that a total thickness of the joint members at the area of overlap is about the same as that of the joint members outside the area of overlap. The difference may preferably be less than 20 %, less than 10 % or less than 5 % of the thickness of the area outside the area of overlap.

Moreover, one or more rotation guide portions 212, 213, 214; 222, 223, 224 may be provided. In the illustrated example, there is provided an outer guide portion 212, 213; 222, 223 and a central guide portion 214; 224.

The central guide portion 214, 224 may be formed by a protrusion 214 in one of the recesses 211 and a depression or through hole 224 in the other one of the recesses 221.

The outer guide portion may be formed by an arched portion 212, 222 of the transition between the recess 211, 221 and the non-overlapping portion of the joint member. This arched portion may be formed so as to fit with a distal portion 213, 223 of the other one of the joint members.

A joint lock (not shown) may be provided with a view to preventing the joint members from separating in the direction along the axis of rotation. Such joint lock may be provided by e.g. a fastener attaching a washer to the protrusion, the washer being larger than the corresponding recess of the other joint member.

Fig. 4 schematically illustrates a second joint concept. This concept resembles the one in fig. 3, but with one of the members 310 providing a slot 313 between a pair of edge portions 311, 312 and the other one (not shown) providing a tongue that is to be received in the slot, together with a pin or other joint lock (not shown) that may provide an axle about which the other joint member (not shown) may rotate.

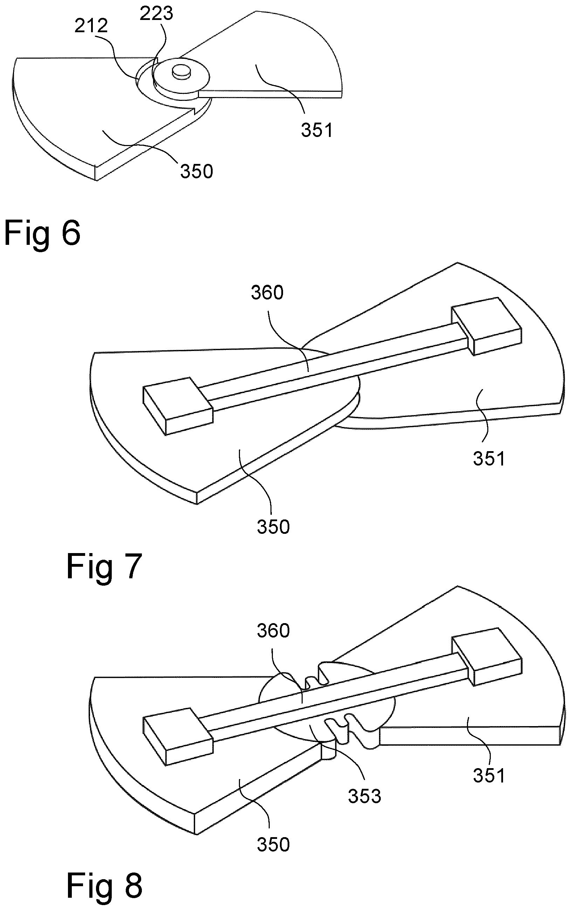

Fig. 6 illustrates a further design, wherein the arched portion 212 of one of the joint members is further spaced from the distal portion 223 of the other joint member, such that a gap is formed therebetween. This gap may correspond to at least 1 %, preferably 5 %, 10 % or 15 %, of a length of the overlap area as seen along the direction of the user's arm. The gap may reduce friction.

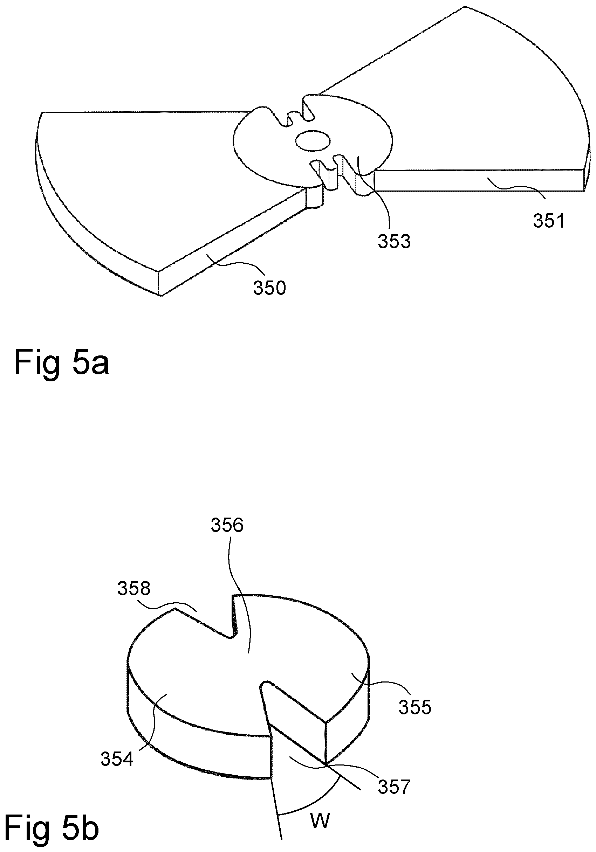

Figs 5a-5b schematically illustrates a third joint concept. This concept is based on a flexible member 535, which may be formed by a separate part that is attached to one or both of the hand support 351 and the wrist support 350, or which may be formed in one piece with one or both of the hand support and the wrist support.

The flexible member 353 may comprise a pair of attachment portions 354, 355, which are separated by a waist portion 356. By designing the member so that its thickness, at and near the waist portion is greater than its width at the waist portion, the member will be prone to bending about the waist portion 356.

The waist portion 356 may be formed by a respective lateral space 357, 358 being provided to extend from an outer lateral edge of the member and inwardly towards the waist portion 356. The lateral spaces 357, 358 may be designed to define a respective central angle W about a bending axis, that will limit the lateral bending of the member.

It is thus possible to control the amount of bending that is to be allowed through the size of the lateral space. Notably, it is possible to provide symmetric or asymmetric bending properties.

With a flexible member, it is possible to provide a bias towards a predetermined relative position of the hand support and the wrist support, in that the flexible member may be elastically flexible, so as to be prone to returning to its original, unloaded position.

The flexible member may be formed of an elastomeric material, which may be foamed. Examples of materials include rubber materials, polyurethane and thermoplastic elastomers.

The flexible member may be releasably attachable to the joint members, such that it is possible to exchange it, e.g. in order to change the angle limitations and/or to change the stiffness of the joint.

Fig. 7 and fig. 8 illustrate embodiments wherein such a biased effect can be provided by means of a spring member 360. The spring member 360 may be combined with the articulated joint designs according to figs 3, 4 and 6. Moreover, the spring member 360 may be used together with the flexible and optionally elastic member 353 of figs 5a-5b, as illustrated in fig. 8.

The spring member 360 may be releasably attached, such that it is possible to change it in order to provide a desired stiffness of the joint.

Finally, and as illustrated in fig. 7, it is possible to base the entire joint on the spring member 360. That is, the spring member may be the device which holds the joint members together. Optionally, an area of overlap between the joint members may be used to limit the bending motion to a single plane.

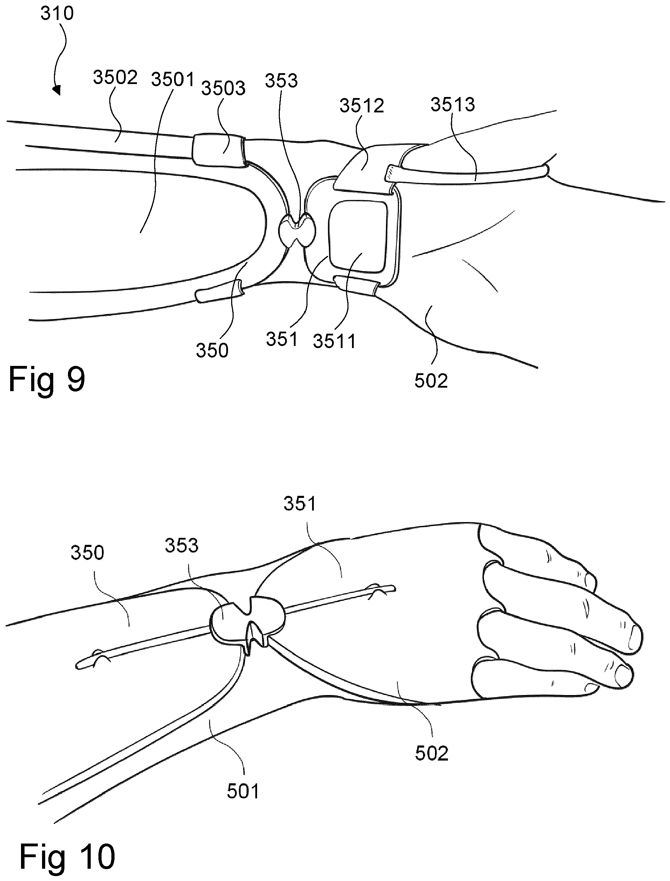

Fig. 9 schematically illustrates an embodiment of a gaming glove worn by a user, as seen from below. In this figure, the wrist support and the hand support are illustrated, as is the joint. While in the illustrated version, the joint is based on figs 5a-5b, it is understood that any one of the joint designs provided herein may be used.

Moreover, in fig. 9, there is illustrated the bearing arrangements 3501, 3511 which are provided on the wrist support 350 and on the hand support 351, as well as the sleeves 3502, 3512 and straps 3503, 3513 that are used to attach the glove to the user's lower arm, to his hand and to his fingers. It is noted that in the embodiment illustrated in fig. 9, the joint is provided on the underside of the device, i.e. below the user's arm and closer to the support surface on which the computer mouse is operated.

Fig. 10 schematically illustrates an embodiment of a gaming glove worn by a user, as seen from above. In this embodiment, the joint 353 is arranged on the upper part of the glove, i.e. on the upper side of the user's hand 502 and wrist 501. The joint may be designed just like in fig. 9.

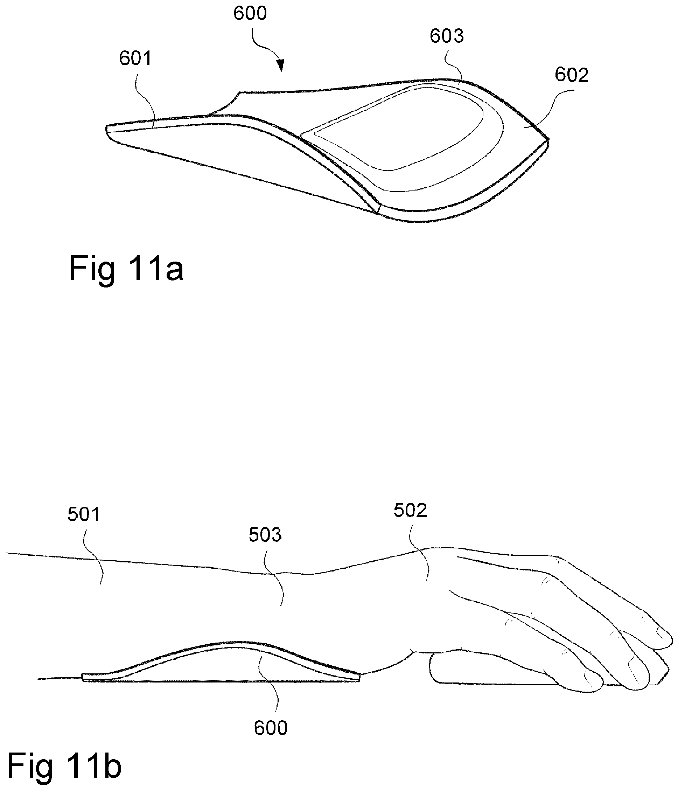

Figs 11a-11b schematically illustrate an ergonomic support member 600, which may be incorporated into the gaming glove. This ergonomic support member is designed to insure that the distal part of the user's radius bone is spaced further from the support surface than it would normally be, such that a desired angle of the user's wrist can be effortlessly maintained.

The support member may comprise a first support surface 601 that is straight or slightly curved in the longitudinal direction of the user's arm and concave as seen in a transversal direction. This first support surface may be intended to support part of the user's lower arm 501. The support member may comprise a summit portion 603, which is concave also in the longitudinal direction, where the user's wrist joint 503 is to be positioned. Finally, the support member may comprise a second support surface 602, that is intended to support the proximal portion of the user's hand 502. When placed on a horizontal surface, the second support surface may present a greater slope than the first support surface, as seen along the longitudinal direction of the user's arm.

The support member 600 may be integrated, such as attached to or formed in one piece with, the wrist support.

The support member 600 may present one or more through holes or openings, such that it will allow air flow to/from the user's arm.

While the device may be used with any type of computer mouse and with any type of program, one specifically suitable use case is for first-person-shooter type games, such as Counter-Strike: Global Offensive^{®}. In such use, a mouse sensitivity may typically be set to less than about 1000 DPI (dots per inch). In such use, the wrist support will be essential in providing friction reduction for large sideways movements of the mouse.

Another specifically suitable use case is for the multiplayer online battle arena type games, such as League Of Legends^{®}. In such use, a mouse sensitivity may typically be set to more than about 2000 DPI (dots per inch). In such use, the hand support will be essential in providing friction reduction for small sideways movements of the mouse.

It may be desirable to provide one or more joint covers. For example, referring to fig. 9, it may be desirable to provide a joint cover on the underside of the joint, to provide a smooth transition between the wrist support 350 and the hand support 351. Such a support may be formed by extending the hand support and the wrist support such that they provide a rotary joint with closely spaced arched edges like illustrated in fig. 4, as opposed to the open joint illustrated in fig. 9. Alternatively, a portion of the hand support and/or of the wrist support may extend below the joint, and optionally overlap the other one of the hand support and/or the wrist support.

For example, it may be desirable to provide a downwardly facing support which extends all the way from the hand support through the wrist support to provide a substantially continuous downwardly facing support surface. Such downwardly facing support surface may thus be continuous as a consequence of an overlap, or it may be articulated.

It may also be desirable to provide padding on the side of the joint that faces the user's wrist joint.

In a further embodiment, the gaming glove may comprise a sleeve that is adapted to provide a compression effect on the user's wrist and/or forearm.

In particular, such sleeve may be adapted to provide a compression on the order of 15-60 mmHG. In various embodiments, the sleeve may be designed to provide a pressures in the following ranges: 20-30 mmHg, 30-40 mmHg, 40-50 mmHg or 50-60 mmHg. In yet further embodiments, the sleeve may be designed to provide pressures in the following ranges: 15-21 mmHg, 23-32 mmHg or 34-46 mmHg.

The sleeve may be provided as a closed sleeve that is pulled onto the user's arm like a sock. Alternatively, the sleeve may be at least partially open, along its length, such that it can be closed by e.g. a zipper, hook-and-loop type fastener or the like.

The sleeve may separate from the remainder of the gaming glove, such that the sleeve is applied to the user's wrist or forearm prior to applying the glove. Alternatively, the sleeve may be attached to or attachable to, the gaming glove.

The sleeve present a length and shape sufficient for it to extend from above the user's elbow, along the forearm and over part of the user's hand. In particular, the sleeve may present a loop, strap or hole adapted for receiving at least one of the user's fingers, for example the user's thumb.

Moreover, at the portion of the gaming glove overlapping the user's hand, it may be desirable to leave the underside of the hand uncovered, such that direct skin contact with the computer mouse is achieved.

Such longer sleeves may be particularly preferred in conjunction with a wrist support designed to extend over at least 60 % of the user's radius bone, as seen along the user's arm, preferably at least 70 %, at least 80 % or at least 90 %. In some embodiments, the wrist support may extend through 100-120 % of the user's radius bone.

Where a separate sleeve is used, the wrist fastening device and the hand fastening device may operate as described above.

As yet another feature, it may be possible to provide an illumination device in or on the gaming glove. For example, an illumination device may be provided to illuminate the base on which the gaming glove/mouse are operated. Such illumination device may comprise a power source, such as a battery, one or more light sources, such as LEDs and optionally one or more light guides. A controller may also be provided, e.g. to turn the illumination on/off and optionally also to change color of the illumination, should the light sources be provided in the form of e.g. RGB enable light sources. The controller may communicate with e.g. a computer, a computer peripheral or a smartphone/tablet. Such communication may be wireless. Thus a user interface may be provided via an app in the smartphone/tablet or in the computer.

## Claims

1. A wearable device for increasing performance, comfort and/or preventing injury of a user of a computer mouse, for use with any type of computer mouse, comprising:
a wrist support (10, 350), having an upper side (11) adapted for receiving the user's wrist and/or lower forearm and an underside (12) adapted to bear against a planar horizontal surface, such as a table,
a wrist fastening device (13, 3503), for securing the wrist to the wrist support (10, 350),
a hand support (20, 351), adapted for receiving a part of the user's hand,
a hand fastening device (30, 3512), for securing the hand to the hand support (20, 352),
wherein the underside (12) presents a bearing arrangement (121a, 121b, 121c, 121d, 121e, 3501, 3511) for reducing friction against the planar surface,
wherein the bearing arrangement comprises a friction reducing surface (121a, 121b, 121c, 121d, 121e, 3501, 3511), arranged on the underside of the wrist support (10, 350), and optionally also on an underside of the hand support (20, 351),
wherein the hand support (20, 351) is movably connected to the wrist support (10, 350) by means of a connection (26, 353) which comprises a rotatable connection (26, 353) by which the hand support (20, 351) is rotatably connected to the wrist support (10, 350), such that the hand support (20, 351) and the wrist support (10, 350) are rotatable relative to each other about an axis (A) extending in a vertical direction, when the wearable device is placed on the horizontal surface.

2. The wearable device as claimed in claim 1, wherein a biasing element (360) is provided to bias the hand support (20, 351) and the wrist support (10, 350) towards a predetermined relative position.

3. The wearable device as claimed in any one of the preceding claims, wherein the hand support (20, 351) is connected to the wrist support (10, 350) by means of a joint (353) which is provided at a position below the user's hand.

4. The wearable device as claimed in claim 1, wherein the wrist fastening device (13, 3503) comprises one or more straps, a cuff or a wristlet.

5. The wearable device as claimed in any one claims 1, 2 or 4, further comprising a hand frame (22, 23, 24, 25), connected to the hand support (20, 351), extending upwardly and/or forwardly and configured to at least laterally interact with the hand.

6. The wearable device as claimed in any one of the preceding claims, further including a support member (600), comprising a support surface that is shaped so as to position a distal portion of the user's radius bone at a higher vertical level than a proximal portion of the user's radius bone, said support member (600) being integrated, such as attached to or formed in one piece with, the wrist support (10, 350).

## Patentansprüche

1. Tragbare Vorrichtung zum Verbessern der Leistung, des Komforts und/oder zum Verhindern von Verletzungen eines Benutzers einer Computermaus für die Verwendung mit jeglicher Art von Computermaus, Folgendes umfassend:
eine Handgelenkstütze (10, 350) mit einer Oberseite (11), die dafür eingerichtet ist, das Handgelenk und/oder den Unterarm des Benutzers aufzunehmen, und einer Unterseite (12), die dafür eingerichtet ist, auf einer ebenen horizontalen Fläche, wie beispielsweise einem Tisch, aufzuliegen,
eine Handgelenk-Befestigungsvorrichtung (13, 3503) zum Sichern des Handgelenks an der Handgelenkstütze (10, 350),
eine Handstütze (20, 351), die dafür eingerichtet ist, einen Teil der Hand des Benutzers aufzunehmen,
eine Handbefestigungsvorrichtung (30, 3512) zum Sichern der Hand an der Handstütze (20, 352),
wobei die Unterseite (12) eine Auflageanordnung (121a, 121b, 121c, 121d, 121e, 3501, 3511) zum Verringern von Reibung an der ebenen Fläche bietet,
wobei die Auflageanordnung eine reibungsmindernde Fläche (121a, 121b, 121c, 121d, 121e, 3501, 3511) umfasst, die an der Unterseite der Handgelenkstütze (10, 350) und optional auch an einer Unterseite der Handstütze (20, 351) angeordnet ist,
wobei die Handstütze (20, 351) mittels einer Verbindung (26, 353), die einen drehbaren Abschnitt (26, 353) umfasst, durch den die Handstütze (20, 351) drehbar mit der Handgelenkstütze (10, 350) verbunden ist, beweglich mit der Handgelenkstütze (10, 350) verbunden ist, so dass die Handstütze (20, 351) und die Handgelenkstütze (10, 350) relativ zueinander um eine Achse (A) drehbar sind, die sich in einer vertikalen Richtung erstreckt, wenn die tragbare Vorrichtung auf der horizontalen Fläche platziert ist.

2. Tragbares Gerät nach Anspruch 1, wobei ein Vorspannelement (360) bereitgestellt ist, um die Handstütze (20, 351) und die Handgelenkstütze (10, 350) hin zu einer festgelegten relativen Position vorzuspannen.

3. Tragbares Gerät nach einem der vorhergehenden Ansprüche, wobei die Handstütze (20, 351) mittels eines Anschlusses (353), der an einer Position unter der Hand des Benutzers bereitgestellt ist, mit der Handgelenkstütze (10, 350) verbunden ist.

4. Tragbares Gerät nach Anspruch 1, wobei die Handgelenk-Befestigungsvorrichtung (13, 3503) ein oder mehrere Bänder, eine Manschette oder ein Armband umfasst.

5. Tragbares Gerät nach einem der Ansprüche 1, 2 oder 4, ferner ein Handgestell (22, 23, 24, 25) umfassend, das mit der Handstütze (20, 351) verbunden ist, sich nach oben und/oder nach vorn erstreckt und dafür gestaltet ist, zumindest seitlich mit der Hand zu interagieren.

6. Tragbares Gerät nach einem der vorhergehenden Ansprüche, ferner ein Stützelement (600) umfassend, das eine Stützfläche umfasst, die derart geformt ist, dass ein distaler Abschnitt des Speichenknochens des Benutzers auf einer höheren vertikalen Ebene als ein proximaler Abschnitt des Speichenknochens des Benutzers positioniert ist, wobei das Stützelement (600) in die Handgelenkstütze (10, 350) integriert ist, wie beispielsweise an ihr angebracht oder einstückig mit ihr gebildet ist.

## Revendications

1. Dispositif portable pour augmenter les performances, le confort et/ou prévenir les blessures d'un utilisateur d'une souris d'ordinateur, destiné à être utilisé avec tout type de souris d'ordinateur, comprenant :
un support de poignet (10, 350), ayant une face supérieure (11) adaptée pour recevoir le poignet et/ou l'avant-bras inférieur de l'utilisateur et une face inférieure (12) adaptée pour s'appuyer contre une surface horizontale plane, telle qu'une table,
un dispositif de fixation de poignet (13, 3503), pour fixer le poignet au support de poignet (10, 350),
un support de main (20, 351) adapté pour recevoir une partie de la main de l'utilisateur,
un dispositif de fixation de main (30, 3512), pour fixer la main au support de main (20, 352),
dans lequel la face inférieure (12) présente un agencement de palier (121a, 121b, 121c, 121d, 121e, 3501, 3511) pour réduire le frottement contre la surface plane,
dans lequel l'agencement de palier comprend une surface de réduction de frottement (121a, 121b, 121c, 121d,121e, 3501, 3511), agencée sur la face inférieure du support de poignet (10, 350), et optionnellement également sur une face inférieure du support de poignet (20, 351),
dans lequel le support de main (20, 351) est raccordé de manière mobile au support de poignet (10, 350) au moyen d'un raccord (26, 353) qui comprend un raccord rotatif (26, 353) par lequel le support de main (20, 351) est raccordé de manière rotative au support de poignet (10, 350), de sorte que le support de main (20, 351) et le support de poignet (10, 350) puissent tourner l'un par rapport à l'autre autour d'un axe (A) s'étendant dans une direction verticale, lorsque le dispositif portable est placé sur la surface horizontale.

2. Dispositif portable selon la revendication 1, dans lequel un élément de sollicitation (360) est prévu pour solliciter le support de main (20, 351) et le support de poignet (10, 350) vers une position relative prédéterminée.

3. Dispositif portable selon une quelconque des revendications précédentes, dans lequel le support de main (20, 351) est raccordé au support de poignet (10, 350) au moyen d'une articulation (353) qui est prévue à une position en dessous de la main de l'utilisateur.

4. Dispositif portable selon la revendication 1, **caractérisé en ce que** le dispositif de fixation de poignet (13, 3503) comprend une ou plusieurs sangles, une manchette ou un bracelet.

5. Dispositif portable selon une quelconque des revendications 1, 2 ou 4, comprenant en outre un cadre de main (22, 23, 24, 25), raccordé au support de main (20, 351) s'étendant vers le haut et/ou vers l'avant et configuré pour interagir au moins latéralement avec la main.

6. Dispositif portable selon une quelconque des revendications précédentes, comprenant en outre un élément de support (600), comprenant une surface de support qui est formée de manière à positionner une partie distale de l'os du radius de l'utilisateur à un niveau vertical plus élevé qu'une partie proximale de l'os du radius de l'utilisateur, ledit élément de support (600) étant intégré, tel que fixé ou formé d'une seule pièce avec, le support de poignet (10, 350).
